Europäisches Patentamt

European Patent Office

Office européen des brevets

(11) Publication number: **0 172 608**
**B1**

# EUROPEAN PATENT SPECIFICATION

(45) Date of publication of patent specification: **20.04.88**

(21) Application number: **85303679.6**

(22) Date of filing: **24.05.85**

(51) Int. Cl.⁴: **C 07 D 487/04,** A 61 K 31/53
// C07D487:04, C07D251:00,
C07D231:00

(54) **Novel triazine derivative, process for preparing same and pharmaceutical preparations containing same.**

(30) Priority: **25.05.84 US 614454**

(43) Date of publication of application:
**26.02.86 Bulletin 86/09**

(45) Publication of the grant of the patent:
**20.04.88 Bulletin 88/16**

(84) Designated Contracting States:
**AT BE CH DE FR GB IT LI LU NL SE**

(56) References cited:
**EP-A-0 122 978**
**US-A-4 279 819**

**CHEMICAL ABSTRACTS, vol. 96, no. 21, 24th
May 1982, page 688, abstract no. 181036s,
Columbus, Ohio, US; K. SENGA et al.:
"Synthesis and enzymic activity of various
substituted pyrazolo1,5-ar-1,3,5-triazines as
adenosine cyclic 3',5'-phosphate
phosphodiesterase inhibitors"**

(73) Proprietor: **BIOMEASURE INC.
11-15 E. Avenue
Hopkinton Massachusetts (US)**

(72) Inventor: **Kim, Sun Hyuk
20 Whitney Street
Chestnut Hill Massachusetts (US)**
Inventor: **Moreau, Jacques-Pierre
159 Westboro Road
Weston Massachusetts (US)**

(74) Representative: **Allam, Peter Clerk et al
LLOYD WISE, TREGEAR & CO. Norman House
105-109 Strand
London WC2R 0AE (GB)**

Courier Press, Leamington Spa, England.

**Description**

This invention relates to a novel triazine derivative that prevents formation of gastric or duodenal ulcers, either by inhibition of gastric acid secretion, or by other mechanisms.

Such a compound may prevent ulcers caused by a variety of stimuli, e.g. histamine, gastrin, food, parasympathetic activity and non-steroidal anti-inflammatory drugs.

The present invention features the compound having anti-ulcer activity and having the name: 4 - [2 - [[[5 - (dimethylamino)methyl - 2 - furanyl]methyl]thio]ethylamino] - 2 - methylthio - 7 - phenylpyrazolo-[1,5 - a] - 1,3,5 - triazine.

Also included within the scope of this invention are the pharmaceutically acceptable salts of this compound.

In addition to inhibiting gastric secretion, this compound and its pharmaceutically acceptable salts, are useful for inhibiting ulcers induced by non-steroidal anti-inflammatory drugs, and can in themselves act as an anti-inflammatory drug. The compound and its salts are also particularly effective in treating mental depression.

When injected or administered in the form of a pill, tablet, capsule or liquid, the compound and its pharmaceutically acceptable salts are potent, non-mutagenic, stable, and will pass through the stomach without losing their effectiveness.

The novel compound of this invention may be prepared by reacting 4 - methoxy - 2 - methylthio - 7 - phenyl - pyrazolo[1,5-a] - 1,3,5 - triazine with 2[[[5 - (dimethylamino)methyl - 2 - furanyl]methyl]thio]-ethanamine, preferably in a solvent such as methanol, whereafter the desired product may be isolated and purified. An illustrative example of this synthesis is given below.

Example

4-[2-[[[5-(dimethylamino)methyl-2-furanyl]methyl]thio]ethylamino]-2-methylthio-7-phenylpyrazolo[1,5-a]-1,3,5-triazine

4.82 g 4-methoxy-2-methylthio-7-phenylpyrazolo[1,5-a]-1,3,5-triazine is suspended in 100 ml of methanol and a solution of 4.3 g. 2 - [[[5 - (dimethyl - amino)methyl - 2 - furanyl]methyl]thio]ethanamine in 10 ml of methanol is added, whereupon the mixture is stirred at room temperature until no further methoxy compound remains. It is necessary to add an additional 1.0 g of amine to complete the reaction. After evaporation of solvent, the residue is chromatographed on silic gel using $CHCl_3$/methanol=25:1 as an eluant. Appropriate fractions are isolated and the solvent is removed *in vacuo* to give an oily product, which is then dissolved in 250 ml of ether and treated with methanol-HCl until no further precipitates form. The partially sticky solid is collected by filtration, washed with ether and small amounts of methanol, and then treated with acetone to give 5.37 g of a colorless solid. TLC (silica gel: $CHCl_3$/OH=9.1) Rf=0.54.

Because NMR indicates 2 molecules of HCl are incorporated, to prepare the final product the dichloride salt is dissolved in methanol, and the solution is adjusted to pH 6 using 1-N-sodium methoxide. After evaporation of the solvent *in vacuo*, the residue is extracted with CHCl, and the CHCl, extracts evaporated *in vacuo* to dryness to yield the monochloride salt, m.p. 172—174°C.

When administered to mammals alone or together with a pharmaceutically acceptable carrier or diluent (eg orally, topically, intravenously, parenterally, nasally or by suppository), the compound of the invention can prevent peptic, duodenal and gastric ulcers. The compound can also be used to treat reflux esophagitis, acute gastritis, and pancreatic insufficiency.

The compound of the invention can inhibit ulcers induced by non-steroidal anti-inflammatory drugs, eg aspirin and indomethacin, without inhibiting their anti-inflammatory and analgesic activity. Thus the compound can be particularly useful in treating or preventing gastric ulcers in patients, e.g. arthritics, who consume non-steroidal anti-inflammatory drugs. The anti-inflammatory action of the compound can even reduce the dosage required of non-steroidal anti-inflammatory drugs. The compound of the invention can also act as an antidepressant.

The compound can be administred to a mammal in a dosage of 2 to 10 mg/kg/day, preferably 4 to 8 mg/ kg/day.

**Claims for the Contracting States: BE CH FR DE GB IT LU NL and SE**

1. 4 - [2 - [[[5 - (dimethylamino)methyl - 2 - furanyl]methyl]thio]ethylamino] - 2 - methylthio - 7 - phenylpyrazolo[1,5 - a] - 1,3,5 - triazine, or a pharmaceutically acceptable salt thereof.

2. A therapeutic composition for preventing or treating ulcers comprising a therapeutically effective amount of a compound according to Claim 1 together with a pharmaceutically acceptable carrier or diluent.

3. A process for preparing a compound according to Claim 1, which is characterized by reacting 4 - methoxy - 2 - methylthio - 7 - phenylpyrazolo[1,5 - a] - 1,3,5 - triazine with 2 - [[[5 - (diamethyl - amino)methyl - 2 - furanyl]methyl]thio]ethanamine, and thereafter isolating and purifying the reaction product.

4. The use of 4 - [2 - [[[5 - (dimethylamino)methyl - 2 - furanyl]methyl]thio]ethylamino] - 2 - methylthio - 7 - phenylpyrazolo[1,5 - a] - 1,3,5 - triazine, or a pharmaceutically acceptable salt thereof, in the manufacture of a therapeutic composition for preventing or treating ulcers.

## 0 172 608

**Claims for the Contacting State: AT**

1. A process for manufacturing a therapeutic composition for preventing or treating ulcers, characterized by iuncorporating in or with a pharmaceutically acceptable carrier or diluent the compound 4 - [2 - [[[5 - (dimethylamino)methyl - 2 - furanyl]methyl]thio]ethylamino] - 2 - methylthio - 7 - phenylpyrazolo[1,5 - a] - 1,3,5 - triazine, or a pharmaceutically acceptable salt thereof.

2. A process for preparing 4 - [2 - [[[5 - (dimethylamino)methyl - 2 - furanyl]methyl]thio]-ethylamino] - 2 - methylthio - 7 - phenylpyrazolo[1,5 - a] - 1,3,5 - triazine, which is characterized by reacting 4 - methoxy - 2 - methylthio - 7 - phenylpyrazolo[1,5 - a] - 1,3,5 - triazine with 2 - [[[5 - (dimethylamino)methyl - 2 - furanyl]methyl]thio]ethanamine, and thereafter isolating and purifying the reaction product.

**Patentansprüche für die Vertragsstaaten: BE CH FR DE GB IT LU NL SE**

1. 4 - (2 - (((5 - (Dimethylamino)methyl - 2 - furanyl)methyl)thio)äthylamino] - 2 - methylthio - 7 - phenylpyrazolo(1,5 - a) - 1,3,5 - triazin oder ein pharmazeutisch verträgliches Salz desselben.

2. Therapeutische Zusammensetzung zur Verhütung oder Behandlung von Geschwüren umfassend eine therapeutisch wirksame Menge einer Verbindung nach Anspruch 1 zusammen mit einem pharmazeutisch verträglichen Träger- oder Verdünnungsmittel.

3. Verfahren zur Herstellung einer Verbindung nach Anspruch 1, dadurch gekennzeichnet, daß 4 - Methoxy - 2 - methylthio - 7 - phenylpyrazolo(1,5-a) - 1,3,5 - triazin mit 2 - (((5 - (Dimethylamino)-methyl - 2 - furanyl)methyl)thio)äthanamin umgesetzt wird und das Reaktionsprodukt darauf isoliert und gereinigt wird.

4. Verwendung von 4 - (2(((5 - (Dimethylamino)methyl - 2 - furanyl)methyl)thio)äthylamino] - 2 - methylthio - 7 - phenyl - pyrazolo(1,5 - a) - 1,3,5 - triazin oder eines pharmazeutisch verträglichen Salzes desselben bei der Herstellung einer therapeutischen Zusammensetzung für die Verhütung oder Behandlung von Geschwüren.

**Patentansprüche für den Vertragsstaat: AT**

1. Verfahren zur Herstellung einer therapeutischen Zusammensetzung für die Verhütung oder Behandlung von Geschwüren, dadurch gekennzeichnet, daß die Verbindung 4 - (2(((5 - (Dimethylamino)-methyl - 2 - furanyl)methyl)thio)äthylamino] - 2 - methylthio - 7 - phenylpyrazolo(1,5 - a) - 1,3,5 - triazin oder ein pharmazeutisch verträgliches Salz derselben in diese allein oder zusammen mit einem pharmazeutisch verträglichen Träger- oder Verdünnungsmittel eingebracht wird.

2. Verfahren zur Herstellung von 4 - (2(((5 - (Dimethylamino)methyl - 2 - furanyl)methyl)thio)-äthylamino] - 2 - methylthio - 7 - phenylpyrazolo(1,5 - a) - 1,3,5 - triazin, dadurch gekennzeichnet, daß 4 - Methoxy - 2 - methylthio - 7 - phenylpyrazolo(1,5-a) - 1,3,5 - triazin mit 4 - (2 - (((5 - (Dimethylamino)methyl - 2 - furanyl)methyl)thio)äthanamin umgesetzt und das Reaktionsprodukt dann isoliert und gereinigt wird.

**Revendications pour les Etats Contactants: BE CH FR DE GB IT LU NL SE**

1. La 4 - [2[[5 - (diméthylamino)méthyl - 2 - furannyl]méthyl]thio]éthylamino] - 2 - méthylthio - 7 - phénylpyrazolo[1,5 - a] - 1,3,5 - triazine, ou un sel acceptable pour l'usage pharmaceutique de ce composé.

2. Une composition thérapeutique pour prévenir ou traiter les ulcères, qui comprend une quantité efficace du point de vue thérapeutique d'un composé selon la revendication 1 avec un véhicule ou diluant acceptable pour l'usage pharmaceutique.

3. Un procédé de préparation d'un composé selon la revendication 1, caractérisé en ce que l'on fait réagir la 4 - méthoxy - 2 - méthylthio - 7 - phénylpyrazolo[1,5-a] - 1,3,5 - triazine avec la 2 - [[[5 - (diméthylamino) - méthyl - 2 - furannyl]méthyl]thio]éthyanamine, après quoi on isole et on purifie le produit de réaction.

4. L'utilisation de la 4 - [2[[5 - (diméthylamino)méthyl - 2 - furannyl]méthyl]thio]éthylamino] - 2 - méthylthio - 7 - phénylpyrazolo[1,5 - a] - 1,3,5 - triazine ou d'un sel acceptable pour l'usage pharmaceutique de ce composé dans la préparation d'une composition thérapeutique pour la prévention ou le traitement des ulcères.

**Revendications pour l'Etat Contractant: AT**

1. Une procédé de préparation d'une composition thérapeutique pour la prévention ou le traitement des ulcères, caractérisé en ce que l'on. incorpore dans un véhicule ou diluant acceptable pour l'usage pharmaceutique ou on combine avec un tel véhicule ou diluant la composé 4 - [2[[5 - (diméthylamino)-méthyl - 2 - furannyl]méthyl]thio]éthylamino] - 2 - méthylthio - 7 - phénylpyrazolo[1,5 - a] - 1,3,5 - triazine ou un sel de ce composé acceptable pour l'usage pharmaceutique.

3

2. Un procédé de préparation de la 4 - [2[[[5 - (diméthylamino)méthyl - 2 - furannyl]méthyl]thio]-éthylamino] - 2 - méthylthio - 7 - phénylpyrazolo[1,5 - a] - 1,3,5 - triazine qui se caractérise en ce que l'on fait réagir la 4 - méthoxy - 2 - méthylthio - 7 - phénylpyrazolo[1,5-a] - 1,3,5 - triazine avec la 2 - [[[5 - (diméthylamino)méthyl - 2 - furannyl]méthyl]thio]éthanamine, puis on isole et on purifie le produit de réaction.